(19) Europäisches Patentamt
European Patent Office
Office européen des brevets

(11) Veröffentlichungsnummer: **0 270 804**
**B1**

# (12) EUROPÄISCHE PATENTSCHRIFT

(45) Veröffentlichungstag der Patentschrift: **26.09.90**

(51) Int. Cl.⁵: **C07C 273/18**, C07C 275/28

(21) Anmeldenummer: **87115753.3**

(22) Anmeldetag: **27.10.87**

## (54) Verfahren zur Herstellung von feinteiligen Harnstoffgruppen enthaltenden Polyisocyanaten.

(30) Priorität: **08.11.86 DE 3638148**

(43) Veröffentlichungstag der Anmeldung:
**15.06.88 Patentblatt 88/24**

(45) Bekanntmachung des Hinweises auf die Patenterteilung:
**26.09.90 Patentblatt 90/39**

(84) Benannte Vertragsstaaten:
**BE DE ES FR GB IT NL**

(56) Entgegenhaltungen:
**EP-A- 0 007 532**
**EP-A- 0 179 343**
**US-A- 3 906 019**

(73) Patentinhaber: **BAYER AG,**
**D-5090 Leverkusen 1 Bayerwerk(DE)**

(72) Erfinder: **Kopp, Richard, Dr., Bilharzstrasse 15,**
**D-5000 Koeln 80(DE)**
Erfinder: **Grögler, Gerhard, Dr.,**
**Von-Diergardt-Strasse 48, D-5090 Leverkusen(DE)**
Erfinder: **Hess, Heinrich, Dr., Koernerstrasse 5,**
**D-5090 Leverkusen(DE)**
Erfinder: **König, Klaus, Dr., Zum Hahnenberg 40,**
**D-5068 Odenthal(DE)**

EP 0 270 804 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft Verfahren zur Herstellung von feinteiligen, festen, Harnstoffgruppen enthaltenden Polyisocyanaten durch Umsetzung von Harnstoffgruppen-freien organischen Polyisocyanaten mit Wasser in Gegenwart einer stabilisierenden Verbindung mit mindestens einer hydrophilen und mindestens einer NCO-reaktiven Gruppe und gegebenenfalls üblicher Emulgatoren und/oder Katalysatoren und/oder Basen.

Verfahren zur Herstellung von festen Harnstoffgruppen enthaltenden Polyisocyanaten durch Umsetzung von Harnstoffgruppen-freien organischen Polyisocyanaten mit einem Überschuß an Wasser als reaktives Medium sind bereits bekannt (u.a. US-PS 39 06 019). Da bei dieser Verfahrensweise die Reaktionsprodukte sofort zusammenbacken und daher nur schwierig auf- und weiterverarbeitbar sind, war man bestrebt, die Reaktionsprodukte in möglichst feinteiliger Form und quantitativer Ausbeute herzustellen. Gemäß der Lehre der DOS 34 38 527 gelingt dies, indem man die Umsetzung in Gegenwart eines hochmolekularen Schutzkolloids wie z.B. Polyacrylat, (Poly)-Cellulose, Polyvinylalkohol usw. durchführt. Trotz ihrer ausgezeichneten Wirksamkeit kann die mehr oder weniger gute Wasserlöslichkeit der hochmolekularen Schutzkolloide zu längeren Produktionsabläufen führen. Außerdem ist nicht auszuschließen, daß sich die Schutzkolloide an der Oberfläche der Harnstoffgruppen enthaltenden Polyisocyanate als Film ablagern, wodurch die Reaktivität der Harnstoffgruppen enthaltenden Polyisocyanate - insbesondere bei längerer Lagerung - beinträchtigt werden kann.

Ziel der vorliegenden Erfindung war es daher, Verfahren zur Herstellung von Harnstoffgruppen enthaltenden Polyisocyanaten zur Verfügung zu stellen, bei denen die Reaktionsprodukte nicht nur feinteilig und in möglichst quantitativer Ausbeute anfallen, sondern auch keinerlei Beeinträchtigung der Reaktivität aufweisen.

Erfindungsgemäß gelingt dieses Verfahren zur Herstellung von feinteiligen, festen, Harnstoffgruppen enthaltenden Polyisocyanaten durch Umsetzung von Wasser mit Harnstoffgruppen-freien organischen Polyisocyanaten in wäßrigen Emulsionen, gegebenenfalls in Gegenwart üblicher Emulgatoren und/oder Katalysatoren und/oder Basen, dadurch gekennzeichnet, daß man die Umsetzung in Gegenwart einer emulsions- und dispersionsstabilisierenden Verbindung der allgemeinen Formel (I)

$(X)_a$-R-$(Y)_b$(I)

durchführt, wobei

X für eine $NH_2$, $NHR^1$ (mit $R^1$ ein $C_1$ - bis $C_{10}$-Alkylrest, der gegebenenfalls eine tertiäre Amingruppe enthält), II, eine OH- oder SH-Gruppe steht,

R für einen aromatischen Rest mit $C_6$ bis $C_{20}$, vorzugsweise $C_6$ bis $C_{10}$, einen heteroaromatischen Rest mit $C_5$ bis $C_{20}$, vorzugsweise $C_6$ bis $C_{10}$, und wenigstens einem Sauerstoff- und/oder Stickstoffatom im Ringsystem, oder für einen aliphatischen Rest mit $C_2$ bis $C_{20}$, vorzugsweise $C_2$ bis $C_{10}$, oder einen cycloaliphatischen Rest mit $C_4$ bis $C_{20}$, vorzugsweise $C_4$ bis $C_{10}$, steht, wobei die genannten Reste mit Halogenatomen (Chlor, Brom) substituiert sein können,

Y für COOH, $COO^-M^+$, wobei M für ein Alkalimetall, Erdalkalimetall, $NH_4$ oder eine NH-$(R^2)_3$-Gruppe mit $R^2$ = Hydroxyalkyl, Alkoxyalkyl, II, Alkyl mit $C_1$ bis $C_{10}$, vorzugsweise $C_1$ bis $C_4$ steht,

$SO_3H$, $SO_3^-M^+$ mit M von der angegebenen Bedeutung, für -$N(R^2)_2$ oder - $\overset{\oplus}{N}H(R^2)_2\ Z^{\ominus}$ mit Z ein Carboxylat- oder ein Sulfonatrest und $R^2$ von der angegebenen Bedeutung

oder für einen Rest der allgemeinen Formel (II)

$$-(CH_2-CH_2-O-)_x-(-CH_2-\underset{\displaystyle R^3}{\underset{|}{C}}HO)_y-R^4 \qquad (II)$$

steht, in der

$R^3$ einen $C_1$-$C_6$, vorzugsweise $C_1$-$C_4$ Alkylrest,

$R^4$ Wasserstoff oder einen $C_1$-$C_6$, vorzugsweise Wasserstoff oder einen $C_1$-$C_4$-Alkylrest bedeutet und

x und y, gleich oder verschieden, für eine ganze Zahl von 0 bis 50, vorzugsweise 0 bis 5 stehen, wobei die Summe von x+y mindestens 1 ist,

und a und b, gleich oder verschieden, für eine ganze Zahl von 1 bis 3, vorzugsweise 1 bis 2 stehen,

mit den Bedingungen, wenn X die Bedeutung OH oder SH hat, daß R kein aromatischer oder heteroaromatischer rest sein darf, und wenn Y einen Rest der allgemeinen Formel (II) darstellt, X nur die Bedeutung von $NH_2$ oder $NHR^2$ haben darf und R eine chemische Bindung darstellt,

oder in Gegenwart von Alkalimetallhydrogensulfit, vorzugsweise Natriumhydrogensulfit, durchführt.

Für die Durchführung des erfindungsgemäßen Verfahrens werden als Harnstoffgruppen-freie Ausgangspolyisocyanate aliphatische, cycloaliphatische, araliphatische, aromatische und heterocyclische Polyisocyanate eingesetzt, wie sie z.B. von W, Siefken in Justus Liebigs Annalen der Chemie, 562, Seiten 75 bis 136, beschrieben werden; beispielsweise solche der Formel

Q(NCO)n ,
in der
n = 2-4, vorzugsweise 2,
und
Q einen aliphatischen Kohlenwasserstoffrest mit 2-18, vorzugsweise 6-10 C-Atomen,
einen cycloaliphatischen Kohlenwasserstoffrest mit 4-15, vorzugsweise 5-10 C-Atomen,
einen aromatischen Kohlenwasserstoffrest mit 6-15, vorzugsweise 6-13 C-Atomen,
oder einen araliphatischen Kohlenwasserstoffrest mit 8-15, vorzugsweise 8-13 C-Atomen,
bedeuten, z.B. Ethylen-diisocyanat, 1,4-Tetra methylendiisocyanat, 1,6-Hexamethylendiisocyanat, 1,12-Dodecandiisocyanat, Cyclobutan-1,3-diisocyanat, Cyclohexan-1,3- une -1,4-diisocyanat sowie beliebige Gemische ihrer Stereo-Isomeren, 1-Isocyanato-3,3,5-trimethyl-5-isocyanatomethyl-cyclohexan (DE-Auslegeschrift 1 202 785, US-Patentschrift 3 401 190), 2,4- und 2,6-Hexahydrotoluylendiisocyanat sowie beliebige Gemische dieser Isomeren, Hexahydro-1,3-und/oder -1,4-phenylendiisocyanat, Perhydro-2,4'-und/oder -4,4'-diphenylmethandiisocyanat, 1,3- und 1,4-Phenylendiisocyanat, 2,4- und 2,6-Toluylendiisocyanat sowie beliebige Gemische dieser Isomeren, Diphenylmethan-2,4'- und/oder -4,4'-und/oder -2,2'-diisocyanat, Naphthylen-1,5-diisocyanat.

Ferner kommen beispielsweise erfindungsgemäß in Frage: Triphenylmethan-4,4', 4"-triisocyanat, Polyphenyl-polymethylenpolyisocyanate, wie sie durch Anilin-Formaldehyd-Kondensation und anschließende Phosgenierung erhalten und z.B. in den GB-Patenschriften 874 430 und 848 671 beschrieben werden, perchlorierte Arylpolyisocyanate, wie sie z.B. in der DE-Auslegeschrift 1 157 601 (US Patentschrift 3 277 138) beschrieben werden, Norbornan-Diisocyanate gemäß US-Patentschrift 3 492 330, Allophanatgruppen aufweisende Polyisocyanate, wie sie z.B. in der GB-Patenschrift 994 890, der BE-Patentschrift 761 626 und der NL-Patentschrift 7 102 524 beschrieben werden, Isocyanuratgruppen aufweisende Polyisocyanate, wie sie z.B. in der US-Patentschrift 3 001 973, in den DE-Patentschriften 1 002 789, 1 222 067 und 1 027 394 sowie in den DE-Offenlegungsschriften 1 929 034 und 2 004 048 beschrieben werden, Urethangruppen aufweisende Polyisocyanate, wie sie z.B. in der BE-Patentschrift 752 261 oder in den US-Patentschriften 3 394 164 und 3 644 457 beschrieben werden, durch Telomerisationsreaktionen hergestellte Polyisocyanate, wie sie z.B. in der US-Patentschrift 3 654 106 beschrieben werden, Estergruppen aufweisende Polyisocyanate, wie sie z.B. in den GB-Patentschriften 965 474 und 1 072 956, in der US-Patentschrift 3 567 763 und in der DE-Patentschrift 1 231 688 genannt werden, Umsetzungsprodukte der obengenannten Isocyanate mit Acetalen gemäß der DE-Patentschrift 1 072 385 und polymere Fettsäureester enthaltende Polyisocyanate gemäß der US-Patentschrift 3 455 883.

Besonders bevorzugt werden in der Regel die technisch leicht zugänglichen Polyisocyanate, z.B. das 2,4- und 2,6-Toluylendiisocyanat sowie beliebige Gemische dieser Isomeren ("TDI"), Polyphenyl-polymethylen-polyisocyanate, wie sie durch Anilin-Formaldehyd-Kondensation und anschließende Phosgenierung hergestellt werden ("rohes MDI") und Urethangruppen, Allophanatgruppen oder Isocyanuratgruppen aufweisenden Polyisocyanate ("modifizierte Polyisocyanate"), insbesondere solche modifizierten Polyisocyanate, die sich vom 2,4- und/oder 2,6-Toluylendiisocyanat bzw. vom 4,4'- und/oder 2,4'-und/oder 2,2'-Diphenylmethandiisocyanat ableiten. Insbesondere bevorzugt ist das Toluylen-2,4-diisocyanat.

Die Polyisocyanate können auch in Form einer (konzentrierten Lösung in einem gegenüber den Polyisocyanaten inerten, mit Wasser nicht mischbaren Lösungsmittel, vorzugsweise aliphatischen oder aromatischen Kohlenwasserstoffen wie z.B. n-Hexan, Cyclohexan, Isooctan, Benzol, Toluol oder Xylol oder hydrophoben Weichmachern eingesetzt werden.

Als stabilisierende, vorzugsweise niedermolekulare Verbindung mit der oben angegebenen allgemeinen Formel I werden vorzugsweise solche eingesetzt, bei denen X und Y folgende Bedeutung haben:

1. $X = NH_2$
$Y = COOH$ wie z.B.
2-, 3- bzw. 4-Aminobenzoesäure, 4-Chloranthranilsäure, 6-Chloranthranilsäure, 3-Amino-4-methylbenzoesäure, 3-Aminozimtsäure, 5-Aminoisophthalsäure, 3-Aminosalicylsäure, 4-Aminosalicylsäure, 5-Aminosalicylsäure, Aminoessigsäure, N-Methylaminoessigsäure, 3-Aminopropansäure, 4-Aminobutansäure, 6-Aminohexansäure, L(+)-2,6-Diaminohexansäure, 11-Aminoundecansäure L(+)-Aminobutandisäure, L(+)-Aminopentandisäure, DL-Methiamin,

2. $X = NH_2$
$Y = COO^{\ominus}M^{\oplus}$ wie z.B.
Alkali- und Erdalkalisalze der unter 1. aufgeführten Carbonsäuren, Salze der unter 1. aufgeführten Carbonsäuren mit tertiären Aminen, z.B. mit Trimethylamin, Triethylamin, Dimethylbenzylamin, Trishydroxyethylamin, Tris[(2-hydroxyethoxy) ethyl]amin, Triethylendiamin,

3. $X = NH_2$
$Y = SO_3H$ wie z.B.
2-, 3- bzw. 4-Aminobenzolsulfonsäure, 3-Amino-6-chlor-benzolsulfonsäure, p-Phenylendiamin-2-sulfonsäure, 4-Aminotoluol-2-sulfonsäure, 5-Aminotoluol-2-sulfonsäure, 2-Aminotoluol-4- sulfonsäure, 3-Amino-benzylsulfonsäure, 4-Aminobenzylsulfonsäure, Naphthionsäure, 2-Aminoethansulfonsäure,

2-Methylaminoethansulfonsäure, 2-Butylaminoethansulfonsäure,

4. X = $NH_2$

Y= $SO_3^{\ominus}M^{\oplus}$ wie z.B.

Alkali-und Erdalkalisalze der unter 3. aufgeführten Sulfonsäuren, Salze der unter 3. aufgeführten Sulfonsäuren mit den unter 2. aufgeführten Aminen,

5. X = $NH_2$ oder $-NHR^1$

Y = $-N(R^2)_2$

1-Amino-3-(dimethylamino)propan, N-Bis (3-dimethylaminoprophyl)amin, N-Methyl-N-(3-aminopropyl)-ethanolamin, N,N-Diethyl-1,3-propandiamin, Bis(3-aminopropyl)-methylamin, 1-Amino-2-diethylaminoethan, 4-Amino-1-diethylaminopentan, 4-Amino-1-dimethylaminopentan, 1-Amino-2-dimethylaminoethan,

6. X = $NH_2$ oder OH

Y = $-\overset{\oplus}{N}H(R^2)_2\ Z^{\ominus}$ wie z.B.

Ammoniumsalze aus mindestens eine OH oder eine primäre und/oder sekundäre und mindestens eine tertiäre Aminogruppe enthaltenden Aminen und einer solchen Menge an Carbon- oder Sulfonsäure, daß mindestens eine NCO-reaktive Gruppe pro Molekül übrig bleibt, vorzugsweise Salze aus N,N-Dimethyl-1,3-propylendiamin und 1 Äquivalent Essigsäure, Propionsäure, Milchsäure, Dimethylolpropionsäure, Methansulfonsäure, Butansulfonsäure, 2-Hydroxy-ethansulfonsäure, oder Salze aus 2-(N,N-Dimethylamino)ethanol, 2-(N,N-Dimethylamino)isopropanol, 2-(N,N-Dimethylaminoethoxy)-ethanol und Carbonsäuren wie Essigsäure, Propionsäure, Benzoesäure, Milchsäure,

7. X = -OH oder -SH

Y = $COO^{\ominus}M^{\oplus}$ wie z.B.

Salze aus Hydroxycarbonsäuren oder Mercaptocarbonsäuren wie Milchsäure, Glykolsäure, Zitronensäure, Dimethylolpropionsäure, Weinsäure, Mercaptoessigsäure und den unter 2. aufgeführten Aminen; oder

8. Natriumhydrogensulfit.

Zur Durchführung des erfindungsgemäßen Verfahrens sollte zunächst, beispielsweise durch einfaches Rühren, eine Lösung aus Wasser, dem erfindungsgemäßen Stabilisator, gegebenefalls einem Emulgator und gegebenefalls einem Katalysator hergestellt werden. Diese Komponenten bilden die wäßrige Phase des Reaktionsgemisches. In diese wäßrige Phase wird dann das Polyisocyanat auf einmal oder kontinuierlich zugesetzt. Es ist aber auch möglich, alle Komponenten auf einmal zu vermischen, solange gewährleistet ist, daß die Emulsion bzw. Dispersion stabil bleibt.

Vorzugsweise verwendet man 0,01 bis 5,0 Gew.-% besonders bevorzugt 0,01 bis 2 Gew.-%, bezogen auf die Menge Polyisocyanat, an stabilisierender Verbindung. Die Menge an organischem Polyisocyanat, bezogen auf das gesamte Reaktionsgemisch, kann zwischen 1 und 75 Gew.-% betragen. Die bevorzugte Menge an organischem Polyisocyanat beträgt 10 bis 30 Gew.-%, besonders bevorzugt 15 bis 25 Gew.-%.

Zur Herstellung der Emulsion verwendet man die üblichen Apparaturen wie Ultraschall-Zerkleinerungsgeräte oder Geräte, an denen die Stoffströme durch Düsen mit hoher Geschwindigkeit gegeneinander, parallel zueinander oder gegen Prallvorrichtungen geschleudert werden. Insbesondere sind jedoch solche Vorrichtungen geeignet, bei denen die Flüssigkeiten unter hoher Beschleunigung und Scherwirkung durch Gitter oder Schlitze geschleudert werden. Die Geräte sind im Handel erhältlich und werden z.B. als Mischsirene bezeichnet. Geräte, die nach dem Rotor-Stator-Prinzip arbeiten, werden bevorzugt eingesetzt.

Die Herstellung der Emulsion erfolgt vorzugsweise bei 10 bis 50°C, besonders bevorzugt bei 23 bis 25°C. Das Polyisocyanat sollte in der wäßrigen Phase mit einer durchschnittlichen Tröpfchengröße von 0,5 bis 200 µm, bevorzugt 0,5 bis 20 µm, vorliegen.

Nach der Herstellung der Emulsion sollte zur Umsetzung des organischen Polyisocyanats mit Wasser eine Reaktionstemperature von 10 bis 80°C, vorzugsweise 20 bis 40°C, vorzugsweise unter Normaldruck, eingehalten werden. Jedoch kann auch unter Zuhilfenahme von geeigneten Vorrichtungen unter einem erhöhten Druck, der sich auch durch das freiwerdende $CO_2$ selbst aufbauen kann, oder unter einem verminderten Druck gearbeitet werden. Die Emulsion bzw. die entstehende Dispersion sollte während der Umsetzung nur mäßig bewegt werden.

Die entstehenden, Harnstoffgruppen enthaltenden Polyisocyanate sind in Wasser schwer löslich und entziehen sich so einer Weiterreaktion der noch vorhandenen NCO-Gruppen mit dem Wasser, so daß im allgemeinen bei den eingesetzten organischen Polyisocyanaten nur eine NCO-Gruppe pro Molekül zur Harnstoffgruppenbildung verbraucht wird. Die NCO-Gehalte der nach dem erfindungsgemäßen Verfahren erhaltenen Produkte liegen in den meisten Fällen nur geringfügig unterhalb des berechneten NCO-Gehalts.

Zur Herstellung der Emulsion können in der wäßrigen oder kontinuierlichen Phase gegebenenfalls übliche, nichtionische, anionische oder kationische oberflächenaktive Emulgatoren mit einem HLB (Hydrophilie-Lipophobie-Balance)-Bereich von 10 bis 18, vorzugsweise 13 bis 16, mitverwendet werden, Der HLB-Wert wird in einer Firmenschrift der Atlas-Chemie, D 4300 Essen, aus dem Jahre 1968, beschrieben. So eignen sich beispielsweise Verbindungen wie Natriumpropyl-naphthalinsulfonat, Polyoxyethylensorbitoleat-laurat, ethoxylierte Nonylphenole, Polyethylenglykolether linearer Alkohole oder Polyethylenglykolester linearer Carbonsäuren. Der Emulgator kann nicht nur der wäßrigen Phase (bevorzugt), sondern auch der organischen Phase beigegeben werden, wobei die üblichen Mengen - 0,01 bis 3,0 Gew.-%, bezogen auf wäßrige Phase - eingehalten werden.

Zur Beschleunigung der Umsetzung des organischen Polyisocyanat mit Wasser können die in der Polyurethanchemie üblichen Katalysatoren, wie z.B. tertiäre Amine (N,N-Dimethylbenzylamin, Triethylamin, Pyridin, Bis(dimethylaminoethyl)ether oder metallorganische Verbindungen (Tributylzinnacetat, Di-n-butyl-zinn-diacetat, Sn(II)-dioctoat, Dibutylzinn-dilaurat) in katalytisch wirksamen, bekannten Mengen eingesetzt werden. Der Katalysatorzusatz erfolgt vorzugsweise zu der wäßrigen Phase, kann aber auch erst nach der Emulsionsbildung erfolgen. Die Katalysatormenge wird dabei vorzugsweise so gewählt, daß die Umsetzung nach ca. 2 bis 8 Stunden beendet ist, und daß die entwickelte Reakionswärme das Gemisch nicht über 35°C erwärmt, so daß eine externe Kühlung entfallen kann.

Die Reaktion zwischem dem organischen Polyisocyanat und Wasser ist an der $CO_2$-Entwicklung zu beobachten. Um Störungen durch Schaumbildung zu vermeiden, kann das $CO_2$ chemisch gebunden werden, indem man die Reaktion bei einem pH$\geqq$7 bis 10 (bevorzugt 8,5 bis 9,5) durch Basenzugabe (z.B. wäßrige Natronlauge) durchführt. Zusätzlich oder alternativ kann auch ein handelsübliches Antischaummittel wie z.B. Tributylphosphat zugegeben werden.

Nach der Beendigung der Umsetzung, die am Aufhören der $CO_2$-Entwicklung zu beobachten ist, wird die Suspension über ein geeignetes Filter abgesaugt, mit Wasser gewaschen, und dann gegebenenfalls mit einem gegenüber Isocyanaten inerten, gegebenenfalls eine gewisse Wasserlöslichkeit besitzenden Lösungsmittel wie z.B. Ethylacetat oder Aceton, nochmals gewaschen und in einem Trockenschrank, vorzugsweise im Vakuum, bei niedrigen Temperaturen (50°C) getrocknet.

Wenn tertiäre Amine als Katalysatoren mitverwendet werden, können diese durch Zugabe einer äquivalenten Säuremenge in Form ihrer Salze vollständig in die wäßrige Phase überführt werden, so daß sie im festen, Harnstoffgruppen enthaltenden Polyisocyanat nach der Aufarbeitung nich mehr vorhanden sind. Auch andere an sich bekannte Verfahren zur Entfernung von mitverwendeten Katalysatoren sind möglich. Gegebenefalls kann die Umsetzung auch durch Inaktivieren des Katalysators zu einem Zeitpunkt beendet werden, der vor dem eigentlichen Reaktionsende liegt.

Bei der Anwendung des erfindungsgemäßen Verfahrens werden Produkte mit nahezu idealer Kugelform erhalten. Die Teilchendurchmesser liegen im allgemeinen bei 1 bis 20 µm.

Der Teilchendurchmesser wird mikroskopisch durch Vergleich mit einer geeichten, in den Strahlengang eingelegten Skala ermittelt.

Die erfindungsgemäß hergestellten feinteiligen, Harnstoffgruppen enthaltenden Polyisocyanate werden vorzugsweise zur Formulierung von Polyurethan-Einkomponentensystemen auf der Basis von Polyaddukt-umhüllten, stabilisierten, festen, feinteiligen Polyisocyanaten retardierter Reaktivität eingesetzt, wie sie z.B. in DE-OS 3 230 757/Ep-A 103 323; DE-OS 3 403 500/EP-A 150 790; DE-OS 3 418 430/EP-A 162 364; DE-OS 3 419 429/EP-A 165 437; DE-OS 3 112 054/EP-PS 62 780; DE-OS 3 343 124/EP-A 145 999 beschrieben sind. Als besonders vorteilhaft erweist sich, daß die feinteiligen Polyisocyanate ohne zusätzliche Mahlung in diesen Einkomponentensystemen eingesetzt werden können.

Allgemeine Beschreibund der Versuchsdurchführung zur Herstellung der Harnstoffgruppen enthaltenden Polyisocyanate

Die angegebene Wassermenge (vorzugswiese demineralisiertes Wasser) wird mit der angegebenen Menge an Stabilisator(lösung), der gegebenenfalls angegebenen Emulgatormenge (vorzugsweise in Form einer verdünnten Lösung), der angegebenen Katalysatormenge und eventuellen weiteren Zusätzen bei Raumtemperatur oder unter leichter Kühlung (ca. 15°C) in einem Becherglas geeigneter Größe homogen vermischt.

Dann wird die abgewogene Menge des Ausgangspolyisocyanats zugesetzt und die zweiphasige Mischung anschließend oder gleichzeitig mit einem Ultraturrax-Rührer (Typ T 45/N der Fa. IKA-Werk, Staufen im Breisgau mit Generator 45 G 6) unter Kühlung intensiv vermischt (Geschwindigkeitsregler auf 1/2 bis 2/3 der vollen Leistung aufgedreht) bis eine stabile Emulsion entstanden ist (i.a. ca. 3 Minuten). Diese wird in einem Reaktionskolben mit Planschliffdeckel überführt und mit einem üblichen Blattrührer weitergerührt.

Falls die wäßrige Phase ohne Katalysator eingesetzt wurde, wird dieser jetzt zugesetzt. Die bei der allmählich beginnenden Reaktion eintretende $CO_2$-Entwicklung wird mit einer Gasuhr verfolgt. Nach Beendigung der $CO_2$-Entwicklung wird gegebenenfalls neutralisiert (z.B. mit 1 N HCl) und die entstandene Suspension abgesaugt, mit Wasser gewaschen und getrocknet.

Die Ausbeute ist praktisch quantitativ, da am Filterkuchen und im Filtrat praktisch kein nicht-umgesetztes Ausgangsisocyanat mehr vorhanden ist.

Beispiel 1

a) Herstellung des harnstoffgruppenhaltigen Polyisocyanats

wäßrige Phase: 700 g Wasser
4,0 g Dispersionsstabilisatorlösung
aus
15 g Glycin
56,2 g Tris-[2-(2-hydroxyethoxy)ethyl]amin
134,4 g Wasser
2,5 g Katalysator Dimethylbenzylamin
org. Phase 150 g 2,4-Toluylendiisocyanat
Produkt: mittlerer Teilchengrößenbereich: 8-14 μm
NCO-Gehalt: 23,62 Gew.-%

b) Herstellung eines Polyharnstoffpolyurethans

100 Teile eines Polyoxypropylenglykolpolyethers mit endständigen aromatischen Amingruppen und einer NH-Zahl von 47,5 (hergestellt nach DOS 2 948 419 durch hydrolyse eines Präpolymers aus 1 Mol Polyoxypropylenglykol, MG 2000, und 2 Mol 2,4-Toluylendiisocyanat)
werden mit
19 Teilen des nach a) hergestellten Harnstoffgruppenenthaltenden Polyisocyanates mit einem starken Rührer mit Zahnkranzrührscheibe intensiv vermischt und anschließend im Wasserstrahlvakuum entgast.
A Dann wird von der Abmischung nach dem in der DOS 3 230 757, S. 49 geschilderten Verfahren eine "Aufdicktemperature" bestimmt und auf einer Metallgießform eine 20 x 20 x 0,3 $cm^3$ große Probeplatte hergestellt (Aushärtung der flüssigen Mischung in 1 Stunde bei 150°C).
B Es wird eine weitere Abmischungen hergestellt, bei der dem aminendständigen Polyoxypropylenpolyether 0,05 Teile (auf 100 Tle Polyether) eines Polyoxypropylenglykols mit endständigen aliphatischen Amingruppen zugesetzt wird, bevor das Harnstoffgruppen enthaltende Polyisocyanat zugemischt wird.
Mit dieser Abmischung wird ebenfalls die "Aufdicktemperatur" bestimmt und eine Probeplatte hergestellt. Tabelle I zeigt die gefundenen Ergebnisse und die mechanischen Werte der hergestellten Probeplatten.

Tabelle 1

| | | A | B |
|---|---|---|---|
| Aufdicktemperatur | | 68 | 82 |
| Mechan. Werte: | | | |
| Zugfestigkeit | | | |
| DIN 53504 (MPa) | | 13,89 | 12,53 |
| 100 % Modul | | | |
| (DIN 53504) (MPa) | | 13,89 | 11,96 |
| Reißdehnung DIN 53504 (%) | | 150 | 200 |
| Weiterreißfestigkeit | | | |
| DIN 53515 (kN/m) | | 30,7 | 29,9 |
| Shore Härte | A | 92 | 90 |
| DIN 53505 | D | 40 | 40 |
| Elastizität | | | |
| DIN 53512 (%) | | 44 | 46 |
| Zeit zum Aushärten einer | | | |
| 3 mm dicken Probe | | | |
| (min'/sec") | | 1'20" | 1'50" |

Die Versuche zeigen, daß die erfindungsgemäß hergestellten Polyisocyanate eine ausgezeichnete Reaktivität aufweisen (siehe Aufdicktemperatur bei Versuch A und B) und gegenüber aliphatischen Polyaminen, die zur Bildung einer retardierenden Polyharnstoffaußenschicht zugesetzt werden, eine (erwünschte) geringe Empfindlichkeit aufweisen.

Beispiel 2

wäßrige Phase: 3500 g Wasser
20 g Stabilisatorlösung
aus
15 g Glycin
29,8 g Triethanolamin
134,4 g Wasser
12,5 g Katalysator Dimethylbenzylamin
org. Phase: 750 g 2,4-Toluylendiisocyanat

Produkt: Teilchengrößenbereich: 6-14 µm
NCO-Gehalt: 24,30 Gew.-%

Beispiel 3

wäßrige Phase: 3500 g Wasser
20 g Stabilisatorlösung
aud
15 g Glycin
29,8 g Triethanolamin
134,4 g Wasser
12,5 g Katalysator Dimethylbenzylamin
org. Phase: 750 g 2,4-Toluylendiisocyanat
Der Katalysator wurde nach Beendigung der Reaktion (Aufhören der $CO_2$-Entwicklung) durch Zusatz der äquivalenten Menge an 1N Salzsäure neutralisiert.
Produkt: mittl. Teilchengrößenbereich: 8-14 µm
NCO-Gehalt: 23,63 Gew.-%

Beispiel 4

wäßrige Phase: 3500 g Wasser
20 g Stabilisatorlösung
aus
15 g Glycin
29,8 g Triethanolamin
134,4 g Wasser
10 g Katalysator 1,4-Diazabicyclooctan
org. Phase: 750 g 2,4-Toluylendiisocyanat
Produkt: mittl. Teilchengrößenbereich: 5-14 µm
NCO-Gehalt: 24,23 Gew.-%

Beispiel 5

wäßrige Phase: 3500 g Wasser
5 g Stabilisatorlösung
aus
60,97 g 2-Aminoethansulfonsäure-Na-salz, 41%ige wäßrige Lösung
118,23 g Wasser
12,5 g Katalysator Dimethylbenzylamin
org. Phase: 750 g 2,4-Toluylendiisocyanat
Produkt: mittl. Teilchengrößenbereich: 5-15 µm
NCO-Gehalt: 23,61 Gew.-%

Beispiel 6

wäßrige Phase: 3500 g Wasser
20 g Stabilisatorlösung
aus
29,05 g 4-Aminobenzoesäure 94,3%ig
29,8 g Triethanolamin
120,4 g Wasser
12,5 g Katalysator (Dimethylbenzylamin)
org. Phase: 750 g 2,4-Toluylendiisocyanat
Produkt: mittl. Teilchengrößenbereich: 4-13 µm
NCO-Gehalt: 23,49 Gew.-%

Beispiel 7

wäßrige Phase: 3500 g Wasser
20 g Stabilisatorlösung
aus
26,2 g 6-Aminohexansäure
29,8 g Triethanolamin
123,2 g Wasser
12,5 g Katalysator Dimethylbenzylamin

org. Phase: 750 g 2,4-Toluylendiisocyanat
Produkt: mittl. Teilchengrößenbereich: 5-15 µm
NCO-Gehalt: 24,18 Gew.-%

Beispiel 8

wäßrige Phase: 3500 g Wasser
20 g Stabilisatorlösung
aus
17,8 g 3-Aminopropansäure
29,8 g Triethanolamin
131,6 g Wasser
12,5 g Katalysator Dimethylbenzylamin
org. Phase: 750 g 2,4-Toluylendiisocyanat
Produkt: Teilchengrößenbereich: 7-14 µm
NCO-Gehalt: 23,48 Gew.-%

Beispiel 9

wäßrige Phase: 700 g Wasser
4 g Stabilisatorlösung
aus
34,6 g 4-Aminobenzolsulfonsäure
29,8 g Triethanolamin
114,8 g Wasser
2,5 g Katalysator Dimethylbenzylamin
org. Phase: 150 g 2,4-Toluylendiisocyanat
Produkt: mittl. Teilchengrößenbereich: 7-14 µm
NCO-Gehalt: 24,10 Gew.-%

Beispiel 10

wäßrige Phase: 3500 g Wasser
20 g Stabilisatorlösung
aus
15 g Glycin
22,4 g 50% wäßr. KOH-Lösung
141,8 g Wasser
12,5 g Katalysator Dimethylbenzylamin
org. Phase: 750 g 2,4-Toluylendiisocyanat
Produkt: Teilchengrößenbereich: 7-14 µm
NCO-Gehalt: 22,99 Gew.-%

Beispiel 11

wäßrige Phase: 3500 g Wasser
20 g Stabilisatorlösung, bestehend aus einer 40%igen Na-Hydrogensulfit-Lösung in Wasser
12,5 g Katalysator Dimethylbenzylamin
org. Phase: 750 g 2,4-Toluylendiisocyanat
Produkt: Teilchengrößenbereich: 6-15 µm
NCO-Gehalt: 24,04 Gew.-%

Beispiel 12

wäßrige Phase: 700g Wasser
2,5 g Stabilisatorlösung
aus
18,4 g Mercaptoessigsäure
29,8 g Triethanolamin
131,0 g Wasser
org. Phase: 150 g
1 g Katalysator Dimethylbenzylamin werden 1,5 h nach der Emulgierung zugesetzt
Produkt: Teilchengrößenbereich: 7-12 µm
NCO-Gehalt: 22,70 Gew.-%

Beispiel 13

wäßrige Phase: 357 g Wasser
2 g Stabilisatorlösung
aus
125 g Milchsäure 80%ig
20,2 g Triethylamin
136,5 g Wasser
org. Phase: 75 g 2,4-Toluylendiisocyanat
1,25 g Katalysator Dimethylbenzylamin werden 30 Minuten nach der Emulgierung zugesetzt
Produkt: Teilchengrößenbereich: 2-12 µm
NCO-Gehalt: 21,00 Gew.-%

Beispiel 14

wäßrige Phase: 3500 g Wasser
20 g Stabilisatorlösung
aus
20,4 g N,N-Dimethyl-1,3-diaminopropan
12,0 g Essigsäure
146,8 g Wasser
12,5 g Katalysator Dimethylbenzylamin
org. Phase: 750 g 2,4-Toluylendiisocyanat
Produkt: Teilchengrößenbereich: 3-12 µm
NCO-Gehalt: 23,06 Gew.-%

Beispiel 15

wäßrige Phase: 3500 g Wasser
20 g Stabilisatorlösung
aus
20,4 g N,N-Dimethyl-1,3-diaminopropan
17,0 g Cyanessigsäure
141,8 g Wasser
12,5 g Katalysator Dimethylbenzylamin
org. Phase: 750 g 2,4-Toluylendiisocyanat
Produkt: Teilchengrößenbereich: 5-15 µm
NCO-Gehalt: 23,72 Gew.-%

Beispiel 16

wäßrige Phase: 3500 g Wasser
20 g Stabilisatorlösung
aus
20,4 g N,N-Dimethyl-1,3-diaminopropan
22,5 g Milchsäure 80%ig
140,8 g Wasser
12,5 g Katalysator Dimethylbenzylamin
org. Phase: 750 g 2,4-Toluylendiisocyanat
Produkt: Teilchengrößenbereich: 5-13 µm
NCO-Gehalt: 23,00 Gew.-%

Beispiel 17

wäßrige Phase: 375 g Wasser
1,0 g 1-Amino-8-hydroxy-3,6-dioxaoctan
12,5 g Katalysator Dimethylbenzylamin
org. Phase: 75 g 2,4-Toluylendiisocyanat
Produkt: Teilchengrößenbereich: 10-18 µm
NCO-Gehalt: 23,30 Gew.-%

Beispiel 18

wäßrige Phase: 3500 g Wasser
14 g Diethanolamin als Stabilisator

12,5 g Katalysator Dimethylbenzylamin
org. Phase: 750 g 2,4-Toluylendiisocyanat
Produkt: Teilchengrößenbereich: 8-15 µm
NCO-Gehalt: 23,30 Gew.-%

Beispiel 19

wäßrige Phase: 3500 g Wasser
3,4 g Ethanolamin als Stabilisator
12,5 g Katalysator Dimethylbenzylamin
org. Phase: 750 g 2,4-Toluylendiisocyanat
Produkt: Teilchengrößenbereich: 9-18 µm
NCO-Gehalt: 24,03 Gew.-%

Beispiel 20

wäßrige Phase: 3500 g Wasser
5 g N,N-Dimethyl-1,3-diaminopropan(1-Amino-3(dimethylamino)propan)
org. Phase: 750 g 2,4-Toluylendiisocyanat
Produkt: Teilchengrößenbereich: 2-15 µm
NCO-Gehalt: 23,36 Gew.-%

**Patentansprüche**

1. Verfahren zur Herstellung von feinteiligen, festen, Harnstoffgruppen enthaltenden Polyisocyanaten durch Umsetzung von Wasser mit Harnstoffgruppen-freien organischen Polyisocyanaten in wäßrigen Emulsionen, gegebenenfalls in Gegenwart üblicher Emulgatoren und/oder Katalysatoren und/oder Basen, dadurch gekennzeichnet, daß man die Umsetzung in Gegenwart einer emulsions- und dispersionsstabilisierenden Verbindung der allgemeinen Formel (I)
$(X)_a\text{-R-}(Y)_b$(I)
durchführt, wobei
X für eine $NH_2$, $NHR^1$ (mit $R^1$ ein $C_1$- bis $C_{10}$-Alkylrest, der gegenbenenfalls eine tertiäre Amingruppe enthält), II, eine OH- oder SH-Gruppe steht,
R für einen aromatischen Rest mit $C_6$ bis $C_{20}$, einen heteroaromatischen Rest mit $C_5$ bis $C_{20}$ und wenigstens einem Sauerstoff- und/oder Stickstoffatom im Ringsystem, oder für einen aliphatischen Rest mit $C_2$ bis $C_{20}$ oder einen cycloaliphatischen Rest mit $C_4$ bis $C_{20}$ steht, wobei die genannten Reste mit Halogenatomen (Chlor, Brom) substituiert sein können,
Y für COOH, $COO^-M^+$, wobei M für ein Alkalimetall, erdalkalimetall, $NH_4$ oder eine $NH\text{-}(R^2)_3$-Gruppe mit $R^2$ = Hydroxyalkyl, Alkoxyalkyl, II, Alkyl mit $C_1$ bis $C_{10}$ steht,
$SO_3H$, $SO_3^-M^+$ mit M von der angegebenen Bedeutung,
für $-N(R^2)_2$ oder - $\overset{\oplus}{N}H(R^2)_2\ Z^{\ominus}$ mit Z ein Carboxylat- oder ein Sulfonatrest und $R^2$ von der angegebenen Bedeutung
oder für einen Rest der allgemeinen Formel (II)

$$-(CH_2\text{-}CH_2\text{-}O\text{-})_x\text{-}(\text{-}CH_2\text{-}\underset{\displaystyle R^3}{\underset{|}{C}}HO)_y\text{-}R^4 \qquad (II)$$

steht, in der
$R^3$ einen $C_1$-$C_6$ Alkylrest,
$R^4$ Wasserstoff oder einen $C_1$-$C_6$ Alkylrest bedeutet und
x und y, gleich oder verschieden, für eine ganze Zahl von 0 bis 50, vorzugsweise 0 bis 5 stehen, wobei die Summe von x+y midestens 1 ist
und a und b, gleich oder verschieden, für eine ganze Zahl von 1 bis 3, vorzugsweise 1 bis 2 stehen,
mit den Bedingungen, wenn X die Bedeutung OH oder SH hat, daß R kein aromatischer oder heteroaromatischer Rest sein darf, und wenn Y einen Rest der allgemeinen Formel (II) darstellt, X nur die Bedeutung von $NH_2$ oder $NHR^2$ haben darf und R eine chemische Bindung darstellt,
oder in Gegenwart von Alkalimetallhydrogensulfit durchführt.

2. Verfahren nach Anspruch 2, dadurch gekennzeichnet, daß man als stabilisierende Verbindung eine Verbindung der allgemeinen Formel (I) einsetzt, in der
X für $NH_2$ und Y für COOH, $COO^{\ominus}M^{\oplus}$, $SO_3H$ oder $SO_3^{\ominus}M^{\oplus}$,

X für $NH_2$ oder OH und Y für - $\overset{\oplus}{N}H(R^2)_2\,Z^{\ominus}$ oder
X für OH oder SH und Y für $COO^{\ominus}M^{\oplus}$ stehen.

3. Verfahren nach Anspruch 1 oder 2, dadurch gekennzeichnet, daß als stabilisierende Verbindung 2-, 3- bzw. 4-Aminobenzoesäure, 4-Chloranthranilsäure, 6-Chloranthranilsäure, 3-Amino-4-methylbenzoesäure, 3-Aminozimtsäure, 5-Aminoisophthalsäure, 3-Aminosalicylsäure, 4-Aminosalicylsäure, 5-Aminosalicylsäure, Aminoessigsäure, N-Methylaminoessigsäure, 3-Aminopropansäure, 4-Aminobutansäure, 6-Aminohexansäure, L(+)-2,6-Diaminohexansäure, 11-Aminoundecansäure L(+)-Aminobutandisäure, L(+)-Aminopentandisäure, DL-Methiamin,
Alkali- und Erdalkalisalze der oben aufgeführten Carbonsäuren, Salze der oben aufgeführten Carbonsäuren mit tertiären Aminen, vorzugsweise mit Trimethylamin, Triethylamin, Dimethylbenzylamin, Trishydroxyethylamin, Tris[(2-hydroxyethoxy)ethyl]amin, Triethylendiamin,
2-, 3- bzw. 4-Aminobenzolsulfonsäure, 3-Amino-6-chlor-benzolsulfonsäure, p-Phenylendiamin-2-sulfonsäure, 4-Aminotoluol-2-sulfonsäure, 5-Aminotoluol-2-sulfonsäure, 2-Aminotoluol-4-sulfonsäure, 3-Amino-benzylsulfonsäure, 4-Aminobenzylsulfonsäure, Naphthionsäure, 2-Aminoethansulfonsäure, 2-Methylaminoethansulfonsäure, 2-Butylaminoethansulfonsäure,
Alkali- und Erdalkalisalze der oben aufgeführten Sulfonsäuren, Salze der oben aufgeführten Sulfonsäuren mit den oben aufgeführten tertieren Aminen,
Ammoniumsalze aus mindestens eine OH und/oder eine primäre und/oder sekundäre Amingruppe und mindestens eine tertiäre Aminogruppe enthaltenden Aminen und einer solchen Menge an Carbon- oder Sulfonsäure, daß mindestens eine NCO reaktive Gruppe pro Molekül übrig bleibt, vorzugsweise Salze aus N,N-Dimethyl-1,3-propylendiamin und 1 Äquivalent Essigsäure, Propionsäure, Milchsäure, Dimethylolpropionsäure, Methansulfonsäure, Butansulfonsäure, 2-Hydroxy-ethansulfonsäure, oder Salze aus 2-(N,N-Dimethylamino)-ethanol, 2-(N,N-Dimethylamino)isopropanol, 2-(N,N-Dimethylaminoethoxy)-ethanol und Carbonsäuren wie Essigsäure, Propionsäure, Benzoesäure, Milchsäure,
Salze aus Hydroxycarbonsäuren oder Mercaptocarbonsäuren, vorzugsweise aus Milchsäure, Glykolsäure, Zitronensäure, Dimethylolpropionsäure, Weinsäure, Mercaptoessigsäure und den oben aufgeführten tertieren Aminen oder
Natriumhydrogensulfit eingesetzt wird.

4. Verfahren nach Anspruch 1 bis 3, dadurch gekennzeichnet, daß die stabilisierende Verbindung in Mengen von 0,01 bis 5,0 Gew.-%, bezogen auf das harnstoffgruppen-freie Polyisocyanat, eingesetzt wird.

## Claims

1. A process for the production of finely divided, solid polyisocyanates containing urea groups by reaction of water with organic polyisocyanates free from urea groups in aqueous emulsions, optionally in the presence of typical emulsifiers and/or catalysts and/or bases, characterized in that the reaction is carried out in the presence of an emulsion- and dispersion-stabilizing compound corresponding to general formula (I)

$$(X)_a\text{–}R\text{–}(Y)_b \quad (I)$$

in which
X is an $NH_2$, $NHR^1$ (with $R^1$ = a $C_{1-10}$ alkyl radical optionally containing a tertiary amino group), II, an OH or SH group,
R is a $C_{6-20}$ aromatic radical, a $C_{5-20}$ heteroaromatic radical containing at least one oxygen and/or nitrogen atom in the ring system or a $C_{2-20}$ aliphatic radical or a $C_{4-20}$ cycloaliphatic radical, the radicals mentioned optionally being substituted by halogen atoms (chlorine, bromine),
Y represents COOH, $COO^-M^+$, where M is an alkali metal, alkaline earth metal, $NH_4$ or an $NH\text{–}(R^2)_3$ group with $R^2$ = hydroxyalkyl, alkoxyalkyl, II, $C_{1-10}$ alkyl, $SO_3H$, $SO_3^-M^+$ where M is as defined above,

$-N(R^2)_2$ or $-\overset{\oplus}{N}(R^2)_2\,Z^{\ominus}$ where Z is a carboxylate or sulfonate group and $R^2$ is as defined above or a radical corresponding to general formula (II)

$$-(CH_2-CH_2-O-)_x-(CH_2-\underset{\substack{|\\R^3}}{C}HO)_y-R^4 \qquad (II)$$

where
$R^3$ is a $C_{1-6}$ alkyl group,
$R^4$ is hydrogen or a $C_{1-6}$ alkyl group and x and y may be the same or different and represent an integer of 0 to 50 and preferably 0 to 5, the sum of x+y being at least 1,
and a and b may be the same or different and represent an integer of 1 to 3 and preferably 1 to 2, with the

proviso where X is OH or SH that R cannot be an aromatic or heteroaromatic radical and, where Y is a radical corresponding to general formula (II), X can only have the meaning $HN_2$ or $NHR^2$ and R is a chemical bond,
or in the presence of alkali metal hydrogen sulfite.

2. A process as claimed in claim 1, characterized in that a compound corresponding to general formula (I) in which

X represents $NH_2$ and Y represents COOH, $COO^{\ominus}M^{\oplus}$, $SO_3H$ or $SO_3{}^{\ominus}M^{\oplus}$,

X represents $NH_2$ or OH and Y represents $-\overset{\oplus}{N}H(R^2)_2\,Z^{\ominus}$ or

X represents OH or SH and Y represents $COO^{\ominus}M^{\oplus}$, is used as the stabilizing compound.

3. A process as claimed in claim 1 or 2, characterized in that the stabilizing compound used is selected from 2-, 3- and 4-aminobenzoic acid, 4-chloroanthranilic acid, 6-chloroanthranilic acid, 3-amino-4-methylbenzoic acid, 3-aminocinnamic acid, 5-aminoisophthalic acid, 3-aminosalicylic acid, 4-aminosalicylic acid, 5-aminosalicylic acid, aminoacetic acid, N-methylaminoacetic acid, 3-aminopropanoic acid, 4-aminobutanoic acid, 6-aminohexanoic acid, L(+)-2,6-diaminohexanoic acid, 11-aminoundecanoic acid, L(+)-aminobutanedioic acid, L(+)-aminopentanedioic acid, DL-methiamine,
alkali metal and alkaline earth metal salts of the carboxylic acids mentioned above, salts of the above-mentioned carboxylic acids with tertiary amines, preferably with trimethylamine, triethylamine, dimethylbenzylamine, trishydroxyethylamine, tris-[(2-hydroxyethyoxy)-ethyl]-amine, triethylenediamine,
2-, 3- and 4-aminobenzenesulfonic acid, 3-amino-6-chlorobenzenesulfonic acid, p-phenylenediamine-2-sulfonic acid, 4-aminotoluene-2-sulfonic acid, 5-aminotoluene-2-sulfonic acid, 2-aminotoluene-4-sulfonic acid, 3-aminobenzylsulfonic acid, 4-aminobenzylsulfonic acid, naphthionic acid, 2-aminoethanesulfonic acid, 2-methylaminoethanesulfonic acid, 2-butylaminoethanesulfonic acid,
alkali metal and alkaline earth metal salts of the sulfonic acids mentioned above, salts of the sulfonic acids mentioned with the tertiary amines mentioned above,
ammonium salts of amines containing at least one OH group and/or a primary and/or secondary amino group and at least one tertiary amino group and such a quantity of carboxylic or sulfonic acid that at least one NCO-reactive group per molecule remains over, preferably salts of N,N-dimethyl-1,3-propylenediamine and 1 equivalent acetic acid, propionic acid, lactic acid, dimethylolpropionic acid, methanesulfonic acid, butanesulfonic acid, 2-hydroxyethanesulfonic acid or salts of 2-(N,N-dimethylamino)-ethanol, 2-(N,N-dimethylamino)-isopropanol, 2-(N,N-dimethylaminoethoxy)-ethanol and carboxylic acids, such as acetic acid, propionic acid, benzoic acid, lactic acid,
salts of hydroxycarboxylic acids or mercaptocarboxylic acids, preferably of lactic acid, glycolic acid, citric acid, dimethylolpropionic acid, tartaric acid, mercaptoacetic acid and the tertiary amines mentioned above or sodium hydrogen sulfite.

4. A process as claimed in claims 1 to 3, characterized in that the stabilizing compound is used in quantities of 0.01 to 5.0% by weight, based on the polyisocyanate free from urea groups.

**Revendications**

1. Procédé de fabrication de polyisocyanates solides et à fines particules, renfermant des groupements urée, en faisant réagir de l'eau avec des polyisocyanates organiques exempts de groupements urée dans des émulsions aqueuses, éventuellement en présence des émulsifiants et/ou des catalyseurs et/ou des bases habituels, caractérisé en ce que la réaction a lieu en présence d'un composé stabilisant de dispersion et d'émulsion de formule générale (I)

$(X)_a{-}R{-}(Y)_b$ (I)

dans laquelle
X représente un $NH_2$, $NHR^1$ (où $R^1$ est un résidu alkyle de $C_1$ à $C_{10}$, comportant éventuellement un groupement amine tertiaire), II, SH ou OH.

R représente un résidu aromatique de $C_6$ à $C_{20}$, un résidu hétéroaromatique de $C_5$ à $C_{20}$ et comportant au moins un atome d'oxygène et/ou un atome d'azote dans la combinaison cyclique, ou bien un résidu aliphatique de $C_2$ à $C_{20}$, ou un résidu cycloaliphatique de $C_4$ à $C_{20}$, les résidus mentionnés pouvant être substitués par des atomes d'halogène (chlore, brome),
Y représente COOH, $COO^-M^+$, où M est un métal alcalin, un métal alcalino-terreux, $NH_4$ ou un groupement $NH{-}(R^2)_3$ où $R^2$ est un hydroxyalkyle, un alcoxyalkyle, II, un résidu alkyle de $C_1$ à $C_{10}$,
représente $SO_3H$, $SO^{\ominus}M^{\oplus}$ où M possède la signification indiquée,

$-N(R^2)_2$ ou $-\overset{\oplus}{N}H(R^2)_2\,Z^{\ominus}$ où Z est un résidu carboxylate ou sulfonate et $R^2$ possède la signification indiquée.

ou représente un résidu de formule générale (II)

$$-(CH_2-CH_2-O-)_x-(-CH_2-\underset{\underset{R^3}{|}}{C}HO)_y-R^4 \qquad (II)$$

dans laquelle
$R^3$ est un résidu alkyle de $C_1$ à $C_6$,
$R^4$ est de l'hydrogène ou un résidu alkyle de $C_1$ à $C_6$ et
x et y, identiques ou différents, représentent un nombre entier de 0 à 50, de préférence de 0 à 5, la somme x+ y étant au moins égale à 1,
et a et b, identiques ou différents, représentent un nombre entier de 1 à 3, de préférence de 1 à 2,
avec les conditions que si X possède la signification OH ou SH, R ne peut être un résidu aromatique ou hétéroaromatique, et que si Y représente un résidu de la formule générale (II), X ne peut posséder que la signification $NH_2$ ou $NHR^2$ et R représente un composé chimique,
ou a lieu en présence d'hydrogénosulfite de métal alcalin.

2. Procédé selon la revendication 1, caractérisé en ce que l'on utilise comme composé stabilisant un composé de la formule générale (I), dans laquelle
X représente $NH_2$ et Y représente COOH, $COO^{\ominus}M^{\oplus}$, $SO_3H$ ou $SO_3{}^{\ominus}M^{\oplus}$
X représente $NH_2$ ou OH et Y représente $-\overset{\oplus}{N}H(R^2)_2\ Z^{\ominus}$ ou
X représente OH ou SH et Y représente $COO^{\ominus}M^{\oplus}$.

3. Procédé selon la revendication 1 ou 2, caractérisé en ce que l'on utilise comme composé stabilisant les composés suivants: acide 2-, 3- ou 4-aminobenzoïque, acide 4-chloranthranilique, acide, acide 6-chloroanthranilique, acide 3-amino- 4-méthylbenzoïque, acide 3-aminocinnamique, acide 5-aminoisophthalique, acide 3-aminosalicylique, acide 4-aminosalicylique, acide 5-aminosalicylique, acide aminoacétique, acide N-méthylaminoacétique, acide 3-aminopropanoïque, acide 4-aminobutanoïque, acide 6-aminohexanoïque, acide L(+)-2,6-diaminohexanoïque, acide 11-aminoundécanoïque, acide L(+)-aminobutanedioïque, acide L(+)-aminopentanedioïque, DL-méthiamine,
des sels de métaux alcalins et alcalino-terreux des acides carboxyliques énumérés ci-avant, des sels des acides carboxyliques énumérés ci-avant avec des amines tertiaires, de préférence avec triméthylamine, triéthylamine, diméthyl-benzylamine, trishydroxyéthylamine, tris[(2-hydroxyéthoxy)éthyl]amine, triéthylènediamine,
les acides 2-, 3- ou 4-aminobenzènesulfonique, 3-amino-6-chlorobenzènesulfonique, p-phénylènediamine-2-sulfonique, 4-aminotoluène-2-sulfonique, 5-aminotoluène-2-sulfonique, 2-aminotoluène-4-sulfonique, 3-amino-benzylsulfonique, 4-aminobenzylsulfonique, naphtionique, 2-aminoéthanesulfonique, 2-méthylaminoéthanesulfonique, 2-butylaminoéthanesulfonique,
des sels de métaux alcalins et alcalino-terreux des acides sulfoniques énumérés ci-avant, des sels des acides sulfoniques énumérés ci-avant avec les amines tertiaire énumérées ci-avant,
des sels d'ammonium d'amines renfermant au moins un OH ou un groupement amino primaire et/ou secondaire et au moins un groupement amino tertiaire et d'une quantité d'acide carboxylique ou sulfonique telle qu'il reste au moins un groupement NCO-réactif libre par molécule, de préférence des sels de N,N-diméthyl-1,3-propylènediamine et de 1 équivalent d'acide acétique, propanoïque, lactique, diméthylolpropanoïque, méthanesulfonique, butanesulfonique, 2-hydroxy-éthanesulfonique ou des sels de 2-(N,N-diméthylamino)-éthanol, 2-(N,N-diméthylamino)isopropanol, 2-(N,N-diméthylaminoéthoxy)-éthanol et d'acides carboxliques, tels que les acides acétique, propanoïque, benzoïque et lactique,
des sels d'acides hydroxycarboxyliques ou d'acides mercaptocarboxyliques, de préférence des acides lactique, glycolique, citrique, diméthylolpropanoïque, tartrique, et mercaptoacétique et des amines énumérées ci-avant; ou
de l'hydrogénosulfite sodique.

4. Procédé selon les revendications 1 à 3, caractérisé en ce que le composé stabilisant est mis en œuvre en quantité de 0,01 à 5,0% en poids par rapport au polyisocyanate exempt de groupements urée.